(19) Europäisches Patentamt
European Patent Office
Office européen des brevets

(11)  **EP 2 697 188 B1**

(12)  **EUROPÄISCHE PATENTSCHRIFT**

(45) Veröffentlichungstag und Bekanntmachung des Hinweises auf die Patenterteilung:
**11.02.2015  Patentblatt 2015/07**

(51) Int Cl.:
***C07C 29/152*** (2006.01)    ***C07C 31/04*** (2006.01)

(21) Anmeldenummer: **12712940.1**

(22) Anmeldetag: **22.03.2012**

(86) Internationale Anmeldenummer:
**PCT/EP2012/001256**

(87) Internationale Veröffentlichungsnummer:
**WO 2012/139703 (18.10.2012 Gazette 2012/42)**

(54) **VERFAHREN UND ANLAGE ZUR HERSTELLUNG VON METHANOL AUS INERTENREICHEM SYNTHESEGAS**

METHOD AND SYSTEM FOR PRODUCING METHANOL FROM INERT-RICH SYNGAS

PROCÉDÉ ET INSTALLATION DE PRODUCTION DE MÉTHANOL À PARTIR DE GAZ DE SYNTHÈSE RICHE EN ÉLÉMENTS INERTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2011  DE 102011017300**

(43) Veröffentlichungstag der Anmeldung:
**19.02.2014  Patentblatt 2014/08**

(73) Patentinhaber: **Air Liquide Global E&C Solutions Germany GmbH**
**60439 Frankfurt am Main (DE)**

(72) Erfinder:
• HACKEL, Philipp Marius
  **60388 Frankfurt am Main (DE)**
• MORGENROTH, Rainer
  **25856 Hattstedt (DE)**
• BORMANN, Andreas
  **60439 Frankfurt (DE)**
• GRONEMANN, Veronika
  **60439 Frankfurt (DE)**

(56) Entgegenhaltungen:
**EP-A1- 2 281 793    WO-A2-03/042144**
**DE-B- 1 296 133**

## Beschreibung

### Gebiet der Erfindung

Die Erfindung betrifft ein Verfahren zur Herstellung von Methanol sowie eine Anlage zur Durchführung dieses Verfahrens. Insbesondere betrifft die Erfindung ein Verfahren zur Umsetzung von Synthesegas mit hohem Anteil an Inertkomponenten zu Methanol, wie es beispielsweise durch Vergasung von Erdgas mit sauerstoffangereicherter Luft oder durch Vergasung von Biomasse oder Kohle mit einem Sauerstoff enthaltenden Gas erhalten wird. Die Erfindung betrifft ferner ein Verfahren zur Umrüstung einer bestehenden Anlage zur Herstellung von Methanol vom Betrieb mit inertenarmem Synthesegas für den Betrieb mit inertenreichem Synthesegas.

### Stand der Technik

Verfahren zur Herstellung von Methanol durch katalytische Umsetzung von Wasserstoff und Kohlenstoffoxide enthaltendem Synthesegas sind der Fachwelt seit langer Zeit bekannt. So werden in Ullmann's Encyclopedia of Industrial Chemistry, Sixth Edition, 1998 Electronic Release, Kapitel "Methanol", Unterkapitel 5.2 "Synthesis" verschiedene Grundverfahren zur Herstellung von Methanol beschrieben.

Ein moderneres, zweistufiges Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt. EP 2281793 offenbart ebenfalls ein Verfahren zur Herstellung von Methanol.

Nachteilig ist bei den beiden oben beschriebenen Verfahren, dass bei der Verarbeitung von Synthesegasen mit hohem Anteil an Inertkomponenten das Kreislaufverhältnis erhöht werden muss, da aufgrund der geringeren Partialdrücke der Reaktanden der Umsatz zu Methanol pro Durchgang durch den Synthesereaktor geringer ist als bei inertenarmem Synthesegas. Dies führt zu einer Erhöhung der benötigten Verdichterleistung, sowie - bei vorgegebener Produktionskapazität für Methanol - zu größeren Dimensionen für Apparate und Rohrleitungen.

Als Inertkomponenten werden dabei einerseits anorganische Gasbestandteile wie Stickstoff oder Edelgase verstanden, die beispielsweise aus der Erzeugung von Synthesegas ausgehend von Erdgas mit entsprechenden Bestandteilen erhalten werden. Solche Erdgase werden beispielsweise aus asiatischen Lagerstätten gewonnen. Auch bei der Vergasung von Erdgasen mit Luft oder sauerstoffangereicherter Luft, wie sie in letzter Zeit vor allem für kleinere Anlagen diskutiert wird, die sich fernab von Luftzerlegungsanlagen zur Sauerstofferzeugung befinden, werden stickstoffreiche Synthesegase erhalten; dies wird beispielsweise in der internationalen Patentanmeldung WO 96/14279 A1 vorgeschlagen. Andererseits wird auch nicht umgesetztes Methan, das bei der Vergasung von Erdgas oder von Kohle im Synthesegasprodukt enthalten sein kann, als ein im Sinne der Methanolsynthese inertes Gas betrachtet, da es in dieser nicht weiter umgesetzt wird.

Die Problematik der Verarbeitung inertenreichen Synthesegases in der Methanolsynthese ist seit längerem bekannt. Es wurden bereits verschiedene technische Lösungsansätze vorgeschlagen, die sich aber aufgrund ihrer Nachteile nicht durchgesetzt haben.

So wird in der Offenlegungsschrift DE 1296133 B vorgeschlagen, das Inertkomponenten wie Stickstoff, Methan oder Argon enthaltende Rohsynthesegas durch eine Xylolwäsche zu behandeln, wodurch deutliche Absenkungen der Gehalte der Inertkomponenten erzielt werden sollen. Nachteilig ist hier, dass die Temperatur des Synthesegases vor Eintritt in die Gaswäsche auf -10 bis -30 °C abgesenkt werden muss, um die Partialdrücke der Inertkomponenten signifikant abzusenken. Damit entsteht ein hoher Exergieverlust. Zudem fällt ein beladenes Absorptionsmittel an, das nachbehandelt werden muss und das die der Methanolsynthese verfahrensfremde Komponente Xylol enthält.

Eine ähnliche technische Lehre ist der deutschen Offenlegungsschrift DE 10156092 A1 zu entnehmen, in der vorgeschlagen wird, jedem katalytischen Reaktionssystem zur Herstellung von Methanol eine Absorptionsstufe vorzuschalten, welche als Absorptionsmittel Methanolsynthesekatalysator enthält, und welche bei einer Temperatur betrieben wird, die unterhalb der für die katalytische Umsetzung zu Methanol liegt. Als Absorptionsmittel wird hierbei ein verfahrenseigener Hilfsstoff herangezogen, allerdings bleiben die beiden bereits ausgeführten Nachteile einer benötigten Temperaturabsenkung sowie einer Nachbehandlung oder Entsorgung des Absorptionsmittels weiterhin bestehen.

Ein Verfahren zur Herstellung von Methanol aus einem aus der autothermen Vergasung von Erdgas erhaltenen Synthesegas, enthaltend jeweils 20 bis 50 % Wasserstoff, Kohlenmonoxid und Methan, beschreibt die Patentanmeldung EP 1819653 A1. Hierbei werden keine besonderen Maßnahmen bezüglich des im Reaktorprodukt des Methanolsynthesereaktors verbleibenden Methans ergriffen, sondern der Wasserstoffgehalt mittels Konvertierung erhöht, sodann der Wasserstoff - gegebenenfalls mit dem ebenfalls erzeugten Kohlendioxid - abgetrennt und dem Methanolsynthesereaktor wieder aufgegeben. Diese Maßnahme erhöht zwar die Partialdrücke der Reaktanden, der Kreislaufstrom bleibt allerdings durch den hohen Inertkomponentenanteil hoch.

Insgesamt ist daher festzustellen, dass bislang keine befriedigende technische Lösung der Aufgabenstellung gefunden wurde, obwohl das Problem, wie dargestellt, bereits seit längerer Zeit besteht. Viele der oben diskutierten Verfahren zielen zudem darauf ab, den Inertkomponenten enthaltenden Spülstrom durch eine entsprechende Behandlung möglichst zu verkleinern.

## Beschreibung der Erfindung

Aufgabe der vorliegenden Erfindung ist es daher, die oben genannten Nachteile zu vermeiden und ein wirtschaftlicheres sowie technisch einfacher durchführbares Verfahren zur Herstellung von Methanol bei Verwendung von inertenreichem Synthesegas als Eduktgas bereitzustellen, das sich insbesondere durch geringen Energiebedarf, kleinere Apparatedimensionen und die Vermeidung prozessfremder Hilfsstoffe auszeichnet.

Die Aufgabe wird gelöst durch ein Verfahren gemäß dem kennzeichnenden Teil des Anspruchs 1 in Zusammenwirken mit den Merkmalen seines Oberbegriffs. Bei dem erfindungsgemäßen Verfahren zur Herstellung von Methanol aus einem Wasserstoff und Kohlenstoffoxide enthaltenden Synthesegas mit hohem Anteil an Inertkomponenten werden im Einzelnen folgende Verfahrensschritte durchlaufen:

- ein erster Synthesegasstrom wird als Einsatzstrom durch einen mindestens einen Methanol-Vorreaktor geleitet, in welchem ein Teil der Kohlenstoffoxide katalytisch mit Wasserstoff zu Methanol umgesetzt wird; das erzeugte Methanol wird abtrennt und so einen zweiter Synthesegasstrom erhalten,
- der zweiten Synthesegasstrom wird durch mindestens einen Methanol-Hauptreaktor geleitet, in welchem ein weiterer Teil der Kohlenstoffoxide katalytisch mit Wasserstoff zu Methanol umgesetzt wird; das erzeugte Methanol wird abtrennt und so ein dritter Synthesegasstrom erhalten,
- der dritten Synthesegasstrom wird in einen vierten Synthesegasstrom und einen fünften Synthesegasstrom aufgeteilt, wobei man den vierten Synthesegasstrom zum Methanol-Hauptreaktor zurückführt und auf diese Weise einen inneren Synthesekreislauf bildet,
- der fünften Synthesegasstrom wird mindestens einer Inertgasabtrennungsstufe zugeführt, wobei ein sechster, an Inertkomponenten abgereicherter Rückführstrom und ein an Inertkomponenten angereicherter Spülstrom erhalten wird,
- der sechste, an Inertkomponenten abgereicherte Rückführstrom wird zu dem oder den Methanol-Hauptreaktoren zurückgeführt und auf diese Weise ein äußerer Synthesekreislauf gebildet.

Überraschenderweise hat sich gezeigt, dass der dem Methanol-Hauptreaktor vorgeschaltete Methanol-Vorreaktor in vorteilhafter Weise mit dem Methanol-Hauptreaktor und der dem Methanol-Hauptreaktor nachgeschalteten Abtrennung inerter Synthesegasbestandteile zusammenwirkt, weil durch die Abreaktion eines Teils des Synthesegases im Vorreaktor zu Methanol die Gasbelastung des Synthesekreislaufs deutlich reduziert wird. Daher können die aus dem Spülgasstrom, dem sog. Purge, abgetrennten Synthesegasbestandteile zurückgeführt werden, ohne dass die benötigte Verdichterleisung, die Abmessungen der eingesetzten Apparate und Rohrleitungen im Synthesekreislauf sowie die im Methanol-Hauptreaktor benötigte Katalysatormenge drastisch ansteigen.

## Bevorzugte Ausgestaltungen der Erfindung

Gemäß einer bevorzugten Ausgestaltung der Erfindung ist vorgesehen, dass die Inertgasabtrennungsstufe eine Druckwechseladsorptionsanlage oder eine Membrananlage umfasst. Auf diese Weise kann ein an Wasserstoff angereicherter und gleichzeitig an Inertkomponenten abgereicherter Gasstrom gewonnen und zum ersten Methanol-Hauptreaktor zurückgeführt werden.

Vorteilhafterweise kann die Inertgasabtrennungsstufe einen Autothermreformer, vorzugsweise gemeinsam mit einer Druckwechseladsorptionsanlage, umfassen.

Der erste Synthesegasstrom kann vorteilhaft durch Vergasung von Erdgas oder Biomasse mit einem Sauerstoff enthaltenden Gas erhalten werden. Alternativ kann der erste Synthesegasstrom auch durch Vergasung von Kohle mit einem Sauerstoff enthaltenden Gas erhalten werden. Im letztgenannten Fall ist das erhaltene Synthesegas häufig besonders methanreich, daher empfiehlt sich es insbesondere in diesem Fall, dass die Inertgasabtrennungsstufe einen Autothermreformer umfasst, da Methan in diesem umgesetzt und somit als Synthesegas gewonnen werden kann.

Die nach dem Methanol-Vorreaktor bzw. nach dem oder den Methanol-Hauptreaktoren abgetrennten Methanolströme können, vorzugsweise gemeinsam, der an sich bekannten Methanol-Produktaufarbeitung zugeführt werden. Diese umfasst üblicherweise einen oder mehrere Destillationsschritte, so dass schließlich Reinmethanol als Produkt gewonnen werden kann.

Der Methanol-Vorreaktor kann in weiterer Ausgestaltung der Erfindung adiabat oder gekühlt, vorzugsweise wassergekühlt, betrieben werden. Beim wassergekühlten Betrieb des Methanol-Vorreaktors kann die Exothermie der Methanolsynthesereaktion für die Dampferzeugung genutzt werden.

Insbesondere bei Beaufschlagung des Methanol-Vorreaktors mit wasserstoffarmen Synthesegasen geringer Stöchiometriezahl SN, die gemäß

$$SN = ((c(H_2) - c(CO_2)) / ((c(CO) + c(CO_2))$$

definiert ist, ist die Methanolsynthesereaktion mit einer hohen Wärmefreisetzung verbunden. Daher ist in weiterer vorteilhafter Ausgestaltung der Erfindung vorgesehen, den Methanol-Vorreaktor mit einer eigenen Synthesegasrückführung auszustatten. Hierzu wird nach Abtrennung des im Methanol-Vorreaktor produzierten Methanols nicht der gesamte den Methanol-Vorreaktor verlassende Synthesegasstrom, sondern lediglich ein Teilstrom zum Methanol-Hauptreaktor geführt und der verbliebene Anteil des Synthesegasstroms vor den Methanol-Vorreaktor zurückgeführt. Auf diese Weise wird die Exothermie der Umsetzung begrenzt, was zu einer verlängerten Lebensdauer bzw. Standzeit des im Methanol-Vorreaktor verwendeten Methanolsynthesekatalysators führt.

Die Methanol-Hauptreaktoren können wassergekühlt, gasgekühlt oder adiabat betrieben werden. Aufgrund der hohen Wärmetönung bei der Methanolsynthese empfiehlt sich der gekühlte Reaktorbetrieb.

Gemäß einer bevorzugten Ausgestaltung der Erfindung sind in den Synthesekreislauf zwei Methanol-Hauptreaktoren vorhanden, wobei der in Strömungsrichtung erste Methanol-Hauptreaktor wassergekühlt und der in Strömungsrichtung zweite Methanol-Hauptreaktor gasgekühlt betrieben wird. Als Kühlgas kann dabei im zweiten Methanol-Hauptreaktor das in den Synthesekreislauf eintretende Synthesegas verwendet werden.

Die Erfindung erstreckt sich auch auf eine Anlage zur Herstellung von Methanol aus einem Wasserstoff und Kohlenstoffoxide enthaltenden Synthesegas mit hohem Anteil an Inertkomponenten, insbesondere zur Durchführung eines Verfahrens nach einem der vorhergehenden Ansprüche, mit einem oder mehreren Methanol-Vorreaktoren, in welchem ein erster Teil der Kohlenstoffoxide katalytisch zu Methanol umgesetzt wird, mit einem oder mehreren Methanol-Hauptreaktoren, in welchem ein weiterer Teil der Kohlenstoffoxide katalytisch zu Methanol umgesetzt wird, einem Abscheider nach dem oder den Methanol-Vorreaktoren zur Abtrennung des Methanols von dem Synthesegas, einem weiteren Abscheider nach dem oder den Methanol-Hauptreaktoren zur Abtrennung weiterer Methanols von dem Synthesegas und Leitungen zur Rückführung eines Synthesegas-Kreislaufstroms zum mindestens einen Methanol-Hauptreaktor. Die erfindungsgemäße Anlage umfasst ferner eine Inertgasabtrennungsstufe sowie Leitungen zum Zuführen von Synthesegas von dem oder den Methanol-Hauptreaktoren zu der Inertgasabtrennungsstufe, Leitungen zum Abführen eines Spülstroms von der Inertgasabtrennungsstufe, sowie Leitungen zur Rückführung eines an Inertkomponenten abgereicherten Rückführstroms zu dem oder den Methanol-Hauptreaktoren.

Die Erfindung betrifft ferner ein Verfahren zur Umrüstung einer bestehenden Anlage zur Herstellung von Methanol mit einem oder mehreren Methanol-Hauptreaktoren innerhalb eines inneren Synthesekreislaufs vom Betrieb mit inertenarmem Synthesegas für den Betrieb mit inertenreichem Synthesegas. Das erfindungsgemäße Umrüstungsverfahren umfasst dabei folgende Maßnahmen:

- Dem ersten Methanol-Hauptreaktor wird ein Methanol-Vorreaktor vorgeschaltet.
- Dem letzten Methanol-Hauptreaktor wird eine Inertgasabtrennungsstufe nachgeschaltet und durch Rückführen eines an Inertkomponenten abgereicherten Rückführstroms zu dem oder den Methanol-Hauptreaktoren mittels entsprechender Leitungen ein äußerer Synthesekreislauf gebildet, wobei sich der oder die Methanol-Vorreaktoren und die Inertgasabtrennungsstufe außerhalb des inneren Synthesekreislaufs befinden.

Ausführungsbeispiele

Weiterbildungen, Vorteile und Anwendungsmöglichkeiten der Erfindung ergeben sich auch aus der nachfolgenden Beschreibung von Ausführungsbeispielen und der Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigen

Figur 1 schematisch eine Anlage zur Herstellung von Methanol nach einem Verfahren gemäß Stand der Technik wie oben beschrieben,

Figur 2 schematisch eine Anlage zur Herstellung von Methanol gemäß einer ersten bevorzugten Ausführungsform der Erfindung,

Figur 3 schematisch eine Anlage zur Herstellung von Methanol gemäß einer zweiten bevorzugten Ausführungsform der Erfindung.

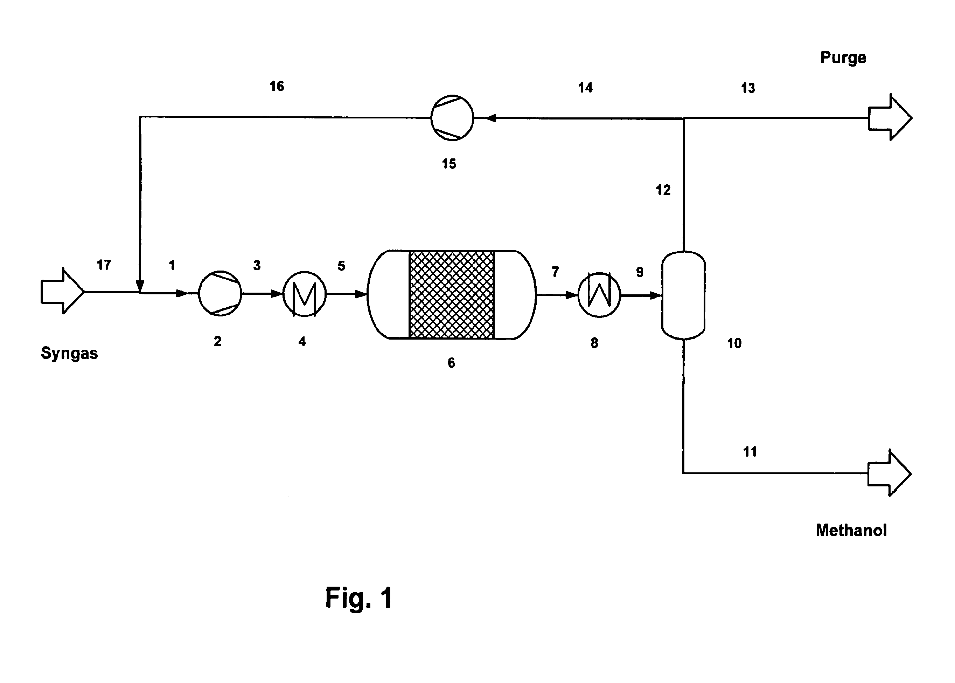

In der in Fig. 1 schematisch und vereinfacht dargestellten Anlage zur Methanolsynthese nach einem Verfahren des Stands der Technik wird ein Wasserstoff und Kohlenstoffoxide enthaltender Synthesegasstrom über Leitung 1 einem Verdichter 2 zugeführt und von diesem auf den Reaktionsdruck von typischerweise 5 bis 10 MPa gebracht. Verdichter 2 und Verdichter 15 können dabei technisch miteinander gekoppelt sein. Der verdichtete Synthesegasstrom wird über Leitung 3 einem Wärmetauscher 4 zugeführt und in diesem auf die Reaktionstemperatur gebracht, wobei zumeist der Wärmetausch gegen den heißen Produktgasstrom aus dem Synthesereaktor erfolgt (nicht dargestellt in Fig. 1). Der vorgeheizte Synthesegasstrom tritt über Leitung 5 in den Methanol-Hauptreaktor 6 ein, wo bei Temperaturen zwischen 200 und 300 °C die teilweise Umsetzung von Wasserstoff mit Kohlenoxiden an einem kupferbasierten Methanolsynthesekatalysator erfolgt, wobei ein Produktgemisch erhalten wird, das Methanol sowie nicht umgesetztes Synthesegas enthält. Es sind verschiedene kupferbasierte Methanolsynthesekatalysatoren im Handel erhältlich, beispielsweise von der Süd-Chemie AG, München. Die Raumgeschwindigkeit im Synthesereaktor beträgt typischerweise 10000 bis 30000 h$^{-1}$. In der schematischen Darstellung der Fig. 1 ist der Methanol-Hauptreaktor einstufig dargestellt; bevorzugt werden jedoch in der praktischen Ausführung mehrere hintereinandergeschaltete Methanol-Hauptreaktoren vorgesehen.

Über Leitung 7 wird das Produktgemisch aus dem Methanol-Hauptreaktor abgeführt. Nach Abkühlung im Wärmetauscher 8, wo eine Abkühlung auf Temperaturen deutlich unterhalb des Taupunktes für Methanol und Wasser, bevorzugt zwischen 30 und 60 °C, erfolgt, gelangt das Produktgemisch über Leitung 9 in den Abscheider 10, wo Methanol als flüssiges, wasserhaltiges Rohmethanol abgetrennt und über Leitung 11 der weiteren Produktaufbereitung zugeführt wird. Das im Abscheider erhaltene Gasprodukt wird über Leitung 12 abgeführt und in einen Spülstrom (Purge), der über Leitung 13 abgeführt wird, und einen Kreislaufstrom, der über Leitung 14 dem Kreislaufverdichter 15 zugeführt wird, aufgetrennt. Über den Spülstrom werden Inertkomponenten aus dem Verfahren ausgeschleust, allerdings nicht in angereicherter Form. Über Leitung 16 wird der Kreislaufstrom zum Synthesereaktor 6 zurückgeführt und auf diese Weise ein Synthesekreislauf gebildet, wobei über Leitung 17 frisches Synthesegas herangeführt und mit dem Kreislaufstrom vereinigt wird. Das Verhältnis der Stoffmengenströme von Kreislaufstrom zu Frischgasstrom wird als Kreislaufverhältnis bezeichnet.

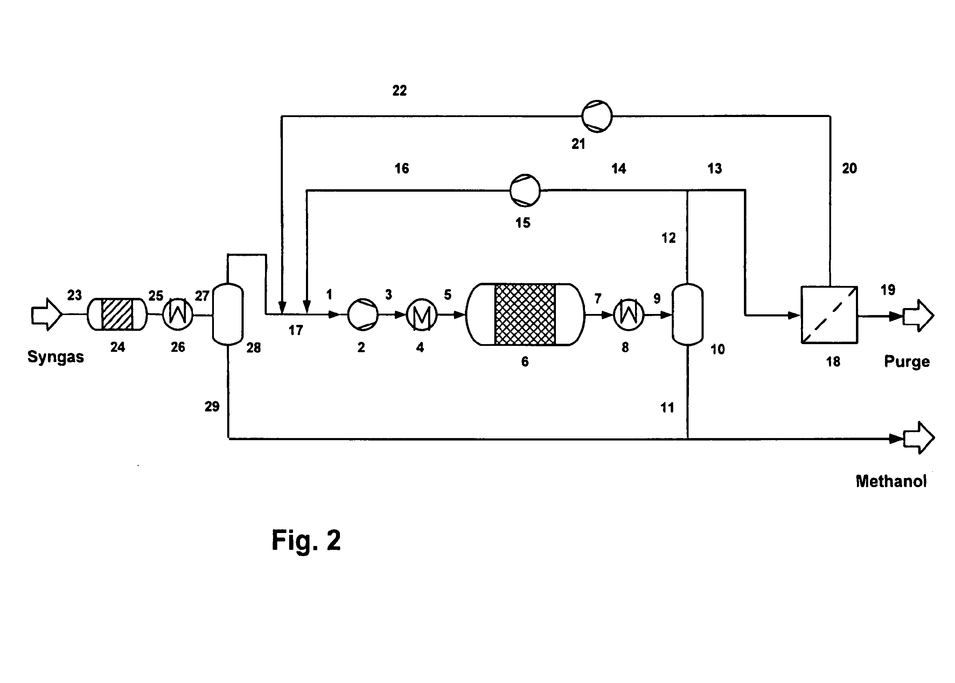

In der in Figur 2 dargestellten Anlage, die eine bevorzugte Ausführungsform der Erfindung darstellt, wird der Synthesegas-Frischgasstrom über Leitung 23 zunächst zu einem Methanol-Vorreaktor 24 geleitet. Es handelt sich hierbei um einen mit körnigem, kupferbasierten Methanolsynthesekatalysator gefüllten adiabaten Festbettreaktor. Gegebenenfalls muss das Synthesegas vor dem Eintritt in den Methanol-Vorreaktor noch aufgeheizt und verdichtet werden, dies ist in Fig. 2 nicht dargestellt. Die Reaktoreintrittstemperatur in den Methanol-Vorreaktor liegt zwischen 190 bis 250 °C, der Reaktionsdruck beträgt typischerweise 5 bis 10 MPa. Die Raumgeschwindigkeit im Methanol-Vorreaktor beträgt typischerweise 5000 bis 15000 h$^{-1}$. Es kann derselbe Katalysatortyp des kupferbasierten Methanolsynthesekatalysators verwendet werden wie im Methanol-Hauptreaktor; dies hat logistische Vorteile beim Betrieb einer Anlage nach dem erfindungsgemäßen Verfahren. Es kann aber auch vorteilhaft sein, einen kupferbasierten Methanolsynthesekatalysator mit im Vergleich zum Methanol-Hauptreaktor höherer Syntheseaktivität zu verwenden. Über Leitung 25 wird das Produktgemisch aus dem Methanol-Vorreaktor abgeführt, im Wärmetauscher 26 auf Temperaturen deutlich unterhalb des Taupunktes für Methanol und Wasser, bevorzugt zwischen 30 und 60 °C, abgekühlt und über Leitung 27 einem Abscheider 28 zugeführt. In diesem wird Methanol als flüssiges, wasserhaltiges Rohmethanol abgetrennt und über Leitung 29 der weiteren Produktaufbereitung zugeführt. Bevorzugt erfolgt dabei die weitere Aufarbeitung gemeinsam mit dem im Abscheider 10 erhaltenen und über Leitung 11 abgeführten Rohmethanol aus dem Methanol-Hauptreaktor.

Wenn der Umsatz des Synthesegases zu Methanol im Methanol-Vorreaktor 24 begrenzt ist, kann es sinnvoll sein, auf den Wärmetauscher 26 und den Abscheider 28 zu verzichten und das nur einen geringen Methanol-Anteil enthaltende Synthesegas direkt über Leitung 17 zum Methanol-Hauptreaktor zu führen.

Als weiterer Unterschied zu dem in Fig. 1 gezeigten Verfahren des Stands der Technik wird in Fig. 2 der Spülstrom über Leitung 13 nicht aus dem Verfahren ausgeleitet, sondern einer Druckwechseladsorptionsanlage 18 zugeführt. Die Funktionsweise und die Betriebsbedingungen einer solchen Anlage sind dem Fachmann an sich bekannt. In dieser wird ein an Wasserstoff abgereicherter und an Inertkomponenten angereicherter Synthesegasstrom erhalten, der über Leitung 19 (Purge) aus dem Verfahren entfernt wird. Ferner wird ein an Wasserstoff angereicherter Synthesegasstrom erhalten, der über Leitung 20, Verdichter 21 und Leitung 22 vor den Methanol-Hauptreaktor zurückgeführt, wodurch ein äußerer Synthesekreislauf gebildet wird.

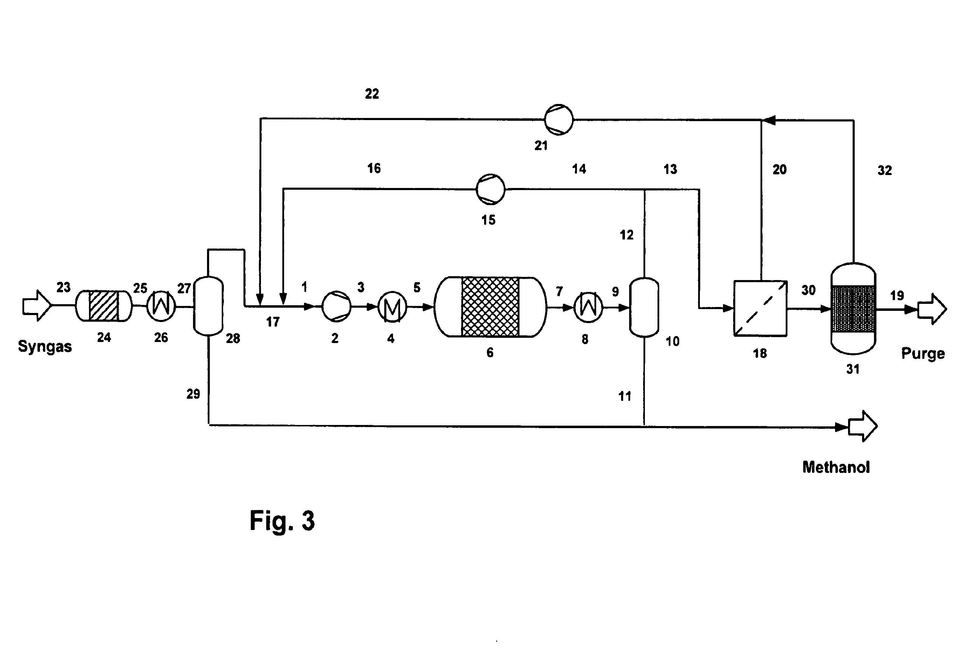

In Figur 3 wird schematisch eine Anlage dargestellt, die eine weitere bevorzugte Ausführungsform der Erfindung darstellt. Diese Ausführungsform wird insbesondere bei dem Einsatz kohlestämmigen Synthesegases für die Methanol-Synthese bevorzugt, das einen vergleichsweise hohen Methangehalt aufweisen kann. So entsteht bei der Kohlevergasung im Festbett ein Synthesegas, das bis zu 60 Vol.% Methan enthalten kann. Bei der in Fig. 3 dargestellten Anlage wird gegenüber Fig. 2 der die Druckwechseladsorptionsanlage 18 verlassende Spülstrom nicht aus dem Verfahren entfernt, sondern über Leitung 30 einem Autothermreformer 31 zugeführt, in dem das im Spülstrom angereicherte Methan zu weiterem Synthesegas umgesetzt wird. Auch die Funktionsweise und die Betriebsbedingungen des Autothermreformers sind dem Fachmann an sich bekannt. Ein Teil des Produktgasstroms des Autothermreformers wird über Leitung 19 als Spülstrom (Purge) aus dem Verfahren entfernt; ein weiterer Teil des Produktgasstroms des Autothermreformers wird über Leitung 32 zum Verdichter 21 geführt und dabei mit dem in Leitung 20 herangeführten Synthesegasstrom vereinigt. In dem Leitungsweg 32 können weitere, in der Fig. nicht gezeigte Aufarbeitungsstufen für das Synthesegas vorgesehen sein. So ist es möglich, den Synthesegas-Teilstrom in Leitung 32 zunächst zur Druckwechseladsorptionsanlage 18 zurückzuführen, um den Wasserstoff abzutrennen und über Leitung 20 zum Methanol-Hauptreaktor zurückzuführen. Der Wasserstoffgehalt im äußeren Synthesekreislauf wird hierdurch nochmals erhöht.

## Gewerbliche Anwendbarkeit

Mit der Erfindung wird somit ein wirtschaftliches Verfahren zur Herstellung von Methanol vorgeschlagen, das sich dadurch auszeichnet, dass auch Synthesegase mit hohen Anteilen an Inertkomponenten verarbeitet werden können. Im Gegensatz zu den im Stand der Technik vorgeschlagenen Verfahren zeichnet sich das erfindungsgemäße Verfahren durch die Abwesenheit prozessfremder oder entsorgungs- bzw. regenerierungsbedürftiger Stoffe wie Absorptionsmittel aus. Als weitere Vorteile sind apparative Einfachheit, kleine Apparategrößen und Rohrleitungsdimensionen, Einsparungen an Katalysator sowie ein geringerer Energiebedarf, beispielsweise für die benötigte Verdichterarbeit, zu nennen.

## Bezugzeichenliste

| | |
|---|---|
| 1 | Leitung |
| 2 | Verdichter |
| 3 | Leitung |
| 4 | Wärmetauscher |
| 5 | Leitung |
| 6 | Methanol-Hauptreaktor(en) |
| 7 | Leitung |
| 8 | Wärmetauscher |
| 9 | Leitung |
| 10 | Abscheider |
| 11 - 14 | Leitung |
| 15 | Verdichter |
| 16 - 17 | Leitung |
| 18 | Druckwechseladsorptionsanlage |
| 19 - 20 | Leitung |
| 21 | Verdichter |
| 22 - 23 | Leitung |
| 24 | Methanol-Vorreaktor(en) |
| 25 | Leitung |
| 26 | Wärmetauscher |
| 27 | Leitung |
| 28 | Abscheider |
| 29 - 30 | Leitung |
| 31 | Autothermer Reformer |
| 32 | Leitung |

## Patentansprüche

1. Verfahren zur Herstellung von Methanol aus einem Wasserstoff und Kohlenstoffoxide enthaltenden Synthesegas mit hohem Anteil an Inertkomponenten, wobei man

- einen ersten Synthesegasstrom als Einsatzstrom durch mindestens einen Methanol-Vorreaktor leitet, in welchem ein Teil der Kohlenstoffoxide katalytisch mit Wasserstoff zu Methanol umgesetzt wird, das erzeugte Methanol abtrennt und so einen zweiten Synthesegasstrom erhält,

- den zweiten Synthesegasstrom durch mindestens einen Methanol-Hauptreaktor leitet, in welchem ein weiterer Teil der Kohlenstoffoxide katalytisch mit Wasserstoff zu Methanol umgesetzt wird, das erzeugte Methanol abtrennt und so einen dritten Synthesegasstrom erhält,

- den dritten Synthesegasstrom in einen vierten Synthesegasstrom und einen fünften Synthesegasstrom aufteilt, wobei man den vierten Synthesegasstrom zum Methanol-Hauptreaktor zurückführt und auf diese Weise einen inneren Synthesekreislauf bildet,

- den fünften Synthesegasstrom mindestens einer Inertgasabtrennungsstufe zuführt, wobei ein sechster, an Inertkomponenten abgereicherter Rückführstrom und ein an Inertkomponenten angereicherter Spülstrom erhalten wird,

- den sechsten, an Inertkomponenten abgereicherten Rückführstrom zu dem oder den Methanol-Hauptreaktoren zurückführt und auf diese Weise einen äußeren Synthesekreislauf bildet.

2. Verfahren nach Anspruch 1, **dadurch gekennzeichnet, dass** die Inertgasabtrennungsstufe eine Druckwechseladsorptionsanlage oder eine Membrananlage umfasst.

3. Verfahren nach Anspruch 1, **dadurch gekennzeichnet, dass** die Inertgasabtrennungsstufe einen Autothermreformer, vorzugsweise gemeinsam mit einer Druckwechseladsorptionsanlage, umfasst.

4. Verfahren nach Anspruch 1 oder 2, **dadurch gekennzeichnet, dass** der erste Synthesegasstrom durch Vergasung von Erdgas oder Biomasse mit einem Sauerstoff enthaltenden Gas erhalten wird.

5. Verfahren nach Anspruch 1 oder 3, **dadurch gekennzeichnet, dass** der erste Synthesegasstrom durch Vergasung von Kohle mit einem Sauerstoff enthaltenden Gas erhalten wird.

6. Verfahren nach einem der vorhergehenden Ansprüche, **dadurch gekennzeichnet, dass** das erhaltene Methanol der weiteren Produktaufarbeitung zugeführt wird.

7. Verfahren nach einem der vorhergehenden Ansprüche, **dadurch gekennzeichnet, dass** der oder die Methanol-Hauptreaktoren wassergekühlt, gasgekühlt oder adiabat betrieben werden.

8. Verfahren nach einem der vorhergehenden Ansprüche, **dadurch gekennzeichnet, dass** zwei Methanol-Hauptreaktoren vorhanden sind, wobei der in Strömungsrichtung erste Methanol-Hauptreaktor wassergekühlt und der in Strömungsrichtung zweite Methanol-Hauptreaktor gasgekühlt betrieben wird.

9. Anlage zur Herstellung von Methanol aus einem Wasserstoff und Kohlenstoffoxide enthaltenden Synthesegas mit hohem Anteil an Inertkomponenten, insbesondere zur Durchführung eines Verfahrens nach einem der vorhergehenden Ansprüche, mit einem oder mehreren Methanol-Vorreaktoren, in welchem ein erster Teil der Kohlenstoffoxide katalytisch zu Methanol umgesetzt wird, mit einem oder mehreren Methanol-Hauptreaktoren, in welchem ein weiterer Teil der Kohlenstoffoxide katalytisch zu Methanol umgesetzt wird, einem Abscheider nach dem oder den Methanol-Vorreaktoren zur Abtrennung des Methanols von dem Synthesegas, einem weiteren Abscheider nach dem oder den Methanol-Hauptreaktoren zur Abtrennung weiteren Methanols von dem Synthesegas, Leitungen zur Rückführung eines Synthesegas-Kreislaufstroms zum mindestens einen Methanol-Hauptreaktoren, **gekennzeichnet durch** eine Inertgasabtrennungsstufe sowie Leitungen zum Zuführen von Synthesegas von dem oder den Methanol-Hauptreaktoren zu der Inertgasabtrennungsstufe, Leitungen zum Abführen eines Spülstroms von der Inertgasabtrennungsstufe, sowie Leitungen zur Rückführung eines an Inertkomponenten abgereicherten Rückführstroms zu dem oder den Methanol-Hauptreaktoren.

10. Verfahren zur Umrüstung einer bestehenden Anlage zur Herstellung von Methanol mit einem oder mehreren Methanol-Hauptreaktoren innerhalb eines inneren Synthesekreislaufs vom Betrieb mit inertenarmem Synthesegas für den Betrieb mit inertenreichem Synthesegas, insbesondere für die Durchführung eines Verfahrens nach Anspruch 1 bis 8, **dadurch gekennzeichnet, dass**

- dem ersten Methanol-Hauptreaktor ein Methanol-Vorreaktor vorgeschaltet wird,

- dem letzten Methanol-Hauptreaktor eine Inertgasabtrennungsstufe nachgeschaltet wird und durch Rückführen eines an Inertkomponenten abgereicherten Rückführstroms zu dem oder den Methanol-Hauptreaktoren ein

## EP 2 697 188 B1

äußerer Synthesekreislauf gebildet wird, wobei sich der oder die Methanol-Vorreaktoren und die Inertgasabtrennungsstufe außerhalb des inneren Synthesekreislaufs befinden.

**Claims**

1. A process for the production of methanol from a synthesis gas containing hydrogen and carbon oxides with a high content of inert components, wherein

   - a first synthesis gas stream is passed as feed stream through at least one methanol pre-reactor, in which a part of the carbon oxides is catalytically converted with hydrogen to obtain methanol, the methanol produced is separated and thus a second synthesis gas stream is obtained,
   - the second synthesis gas stream is passed through at least one methanol main reactor, in which a further part of the carbon oxides is catalytically converted with hydrogen to obtain methanol, the methanol produced is separated and thus a third synthesis gas stream is obtained,
   - the third synthesis gas stream is divided into a fourth synthesis gas stream and a fifth synthesis gas stream, wherein the fourth synthesis gas stream is recirculated to the methanol main reactor and in this way forms an inner synthesis cycle,
   - the fifth synthesis gas stream is supplied to at least one inert gas separation stage, wherein a sixth recirculation stream depleted of inert components and a purge stream enriched in inert components are obtained,
   - the sixth recirculation stream depleted of inert components is recirculated to the methanol main reactor or reactors and in this way forms an outer synthesis cycle.

2. The method according to claim 1, **characterized in that** the inert gas separation stage comprises a pressure swing adsorption system or a membrane system.

3. The method according to claim 1, **characterized in that** the inert gas separation stage comprises an autothermal reformer, preferably jointly with a pressure swing adsorption system.

4. The method according to claim 1 or 2, **characterized in that** the first synthesis gas stream is obtained by gasification of natural gas or biomass with a gas containing oxygen.

5. The method according to claim 1 or 3, **characterized in that** the first synthesis gas stream is obtained by gasification of coal with a gas containing oxygen.

6. The method according to any of the preceding claims, **characterized in that** the methanol obtained is supplied to the further product processing.

7. The method according to any of the preceding claims, **characterized in that** the one or more methanol main reactors are operated in a water-cooled, gas-cooled or adiabatic manner.

8. The method according to any of the preceding claims, **characterized in that** two methanol main reactors are present, wherein the first methanol main reactor in flow direction is operated in a water-cooled manner and the second methanol main reactor in flow direction is operated in a gas-cooled manner.

9. A plant for the production of methanol from a synthesis gas containing hydrogen and carbon oxides with a high content of inert components, in particular for carrying out a process according to any of the preceding claims, with one or more methanol pre-reactors in which a first part of the carbon oxides is catalytically converted to methanol, with one or more methanol main reactors in which a further part of the carbon oxides is catalytically converted to methanol, a separator downstream of the methanol pre-reactor or pre-reactors for separating the methanol from the synthesis gas, a further separator downstream of the methanol main reactor or reactors for separating further methanol from the synthesis gas, conduits for recirculating a synthesis gas cycle stream to the at least one methanol main reactor, **characterized by** an inert gas separation stage as well as conduits for supplying synthesis gas from the methanol main reactor or reactors to the inert gas separation stage, conduits for discharging a purge stream from the inert gas separation stage, and conduits for recirculating a recirculation stream depleted of inert components to the methanol main reactor or reactors.

10. A method for retrofitting an existing plant for the production of methanol with one or more methanol main reactors

8

within an inner synthesis cycle from the operation with synthesis gas low in inerts to the operation with synthesis gas rich in inerts, in particular for carrying out a process according to claims 1 to 8, **characterized in that**

- upstream of the first methanol main reactor a methanol pre-reactor is provided,
- downstream of the last methanol main reactor an inert gas separation stage is provided, and by recirculating a recirculation stream depleted of inert components to the methanol main reactor or reactors an outer synthesis cycle is formed, wherein the methanol pre-reactor or pre-reactors and the inert gas separation stage are located outside the inner synthesis cycle.

**Revendications**

1. Procédé de production de méthanol à partir de gaz de synthèse contenant de l'hydrogène et des oxydes de carbone et ayant une grande proportion de constituants inertes, dans lequel

- on fait passer un premier courant de gaz de synthèse comme courant de charge dans au moins un préréacteur de méthanol, dans lequel on transforme une partie des oxydes de carbone catalytiquement par de l'hydrogène en du méthanol, on sépare le méthanol produit et on obtient ainsi un deuxième courant de gaz de synthèse,
- on fait passer le deuxième courant de gaz de synthèse dans au moins un réacteur principal de méthanol, dans lequel on transforme une autre partie des oxydes de carbone catalytiquement par de l'hydrogène en du méthanol, on sépare le méthanol produit et on obtient ainsi un troisième courant de gaz de synthèse,
- on subdivise le troisième courant de gaz de synthèse en un quatrième courant de gaz de synthèse et en un cinquième courant de gaz de synthèse, on retourne le quatrième courant de gaz de synthèse au réacteur principal de méthanol et on forme ainsi un circuit intérieur de synthèse.
- on envoie le cinquième courant de gaz de synthèse à au moins un étage de séparation de gaz inerte en obtenant un sixième courant de recyclage appauvri en constituants inertes et un courant de balayage enrichi en constituants inertes,
- on retourne le sixième courant de recyclage appauvri en constituants inertes au réacteur principal de méthanol ou aux réacteurs principaux de méthanol et on forme ainsi un circuit extérieur de synthèse.

2. Procédé suivant la revendication 1, **caractérisé en ce que** l'étage de séparation de gaz inerte comprend une installation d'adsorption par alternance de pression ou une installation à membrane.

3. Procédé suivant la revendication 1, **caractérisé en ce que** l'étage de séparation de gaz inerte comprend un reformeur autothermique, de préférence ensemble avec une installation d'adsorption par alternance de pression.

4. Procédé suivant la revendication 1 ou 2, **caractérisé en ce qu'**on obtient le premier courant de gaz de synthèse par gazéification de gaz naturel ou de biomasse par un gaz contenant de l'oxygène.

5. Procédé suivant la revendication 1 ou 3, **caractérisé en ce qu'**on obtient le premier courant de gaz de synthèse par gazéification du charbon par un gaz contenant de l'oxygène.

6. Procédé suivant l'une des revendications précédentes, **caractérisé en ce qu'**on envoie le méthanol obtenu à un autre traitement du produit.

7. Procédé suivant l'une des revendications précédentes, **caractérisé en ce qu'**on fait fonctionner le réacteur principal de méthanol ou les réacteurs principaux de méthanol en les refroidissant par de l'eau, en les refroidissant par du gaz ou adiabatiquement.

8. Procédé suivant l'une des revendications précédentes, **caractérisé en ce qu'**il y a deux réacteurs principaux de méthanol et on fait fonctionner le premier, dans le sens du courant, réacteur principal de méthanol en le refroidissant par de l'eau et le second, dans le sens du courant, réacteur principal de méthanol en le refroidissant par du gaz.

9. Installation de production de méthanol à partir d'un gaz de synthèse contenant de l'hydrogène et des oxydes de carbone et ayant une grande proportion de constituants inertes, notamment pour effectuer un procédé suivant l'une des revendications précédentes, comprenant un ou plusieurs préréacteurs de méthanol, dans lequel une première partie des oxydes de carbone est transformée catalytiquement en méthanol, comprenant un ou plusieurs réacteurs principaux de méthanol, dans lequel une autre partie des oxydes de carbone est transformée catalytiquement en méthanol, un séparateur, après le ou les préréacteurs de méthanol, pour séparer le méthanol du gaz de synthèse, un autre séparateur, après le ou les réacteurs principaux de méthanol, pour séparer davantage de méthanol du gaz de synthèse, des conduits de renvoi d'un courant de circuit de gaz de synthèse à au moins un réacteur principal de méthanol, **caractérisée par** un étage de séparation de gaz inerte, ainsi que par des conduits d'envoi de gaz de synthèse du réacteur principal de méthanol ou des réacteurs principaux de méthanol à l'étage de séparation de gaz inerte, des conduits d'évacuation d'un courant de balayage de l'étage de séparation de gaz inerte, ainsi que des conduits de renvoi d'un courant de recyclage appauvri en gaz inerte au réacteur principal de méthanol ou aux réacteurs principaux de méthanol.

10. Procédé de transformation d'une installation existante de production de méthanol ayant un réacteur principal de méthanol ou plusieurs réacteurs principaux de méthanol au sein d'un circuit intérieur de synthèse fonctionnant avec du gaz de synthèse pauvre en inertes pour le fonctionnement avec du gaz de synthèse riche en inertes, notamment pour la mise en oeuvre d'un procédé suivant les revendications 1 à 8, **caractérisé en ce que**

- on monte un préréacteur de méthanol en amont du premier réacteur principal de méthanol,
- on monte un étage de séparation de gaz inerte en aval du dernier réacteur principal de méthanol et, en renvoyant un courant de renvoi appauvri en constituants inertes au réacteur principal de méthanol ou aux réacteurs principaux de méthanol, on forme un circuit extérieur de synthèse, le préréacteur ou les préréacteurs de méthanol et l'étage de séparation de gaz inerte se trouvant à l'extérieur du circuit intérieur de synthèse.

**Fig. 1**

EP 2 697 188 B1

Fig. 2

Syngas

Methanol

Purge

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0790226 B1
- DE 2934332 A1
- EP 1016643 A1
- EP 2281793 A
- WO 9614279 A1
- DE 1296133 B
- DE 10156092 A1
- EP 1819653 A1

**In der Beschreibung aufgeführte Nicht-Patentliteratur**

- Electronic Release. Ullmann's Encyclopedia of Industrial Chemistry. 1998